# EUROPÄISCHE PATENTSCHRIFT

(11) **EP 1 343 729 B1**
(45) Veröffentlichungstag und Bekanntmachung des Hinweises auf die Patenterteilung: **03.05.2006**
(21) Anmeldenummer: 01995598.8
(22) Anmeldetag: 13.12.2001
(51) Int. Cl.: C02F 3/34, C12N 1/00, C12N 1/20

(54) **MIKROBIOLOGISCHE ZUSAMMENSETZUNG**
MICROBIOLOGICAL COMPOSITION
COMPOSITION MICROBIOLOGIQUE

(30) Priorität: 18.12.2000 DE 10062812
(43) Veröffentlichungstag der Anmeldung: 17.09.2003
(73) Patentinhaber: Georg Fritzmeier GmbH & Co., D-85655 Grosshelfendorf (DE)
(72) Erfinder: UPHOFF, Christian, 83229 Aschau (DE)
(74) Vertreter: Winter, Brandl, Fürniss, Hübner Röss, Kaiser, Polte Partnerschaft Patent- und Rechtsanwaltskanzlei
(86) Internationale Anmeldenummer: PCT/DE2001/004713
(87) Internationale Veröffentlichungsnummer: WO 2002/049971

(56) Entgegenhaltungen:
- EP-A- 0 372 520
- DE-C- 3 912 420
- US-A- 5 707 856
- PATENT ABSTRACTS OF JAPAN vol. 1998, no. 05, 30. April 1998 (1998-04-30) & JP 10 008525 A (SAYAMA YUICHI), 13. Januar 1998 (1998-01-13)
- PATENT ABSTRACTS OF JAPAN vol. 1997, no. 07, 31. Juli 1997 (1997-07-31) & JP 09 075983 A (NIPPON KENTETSU CO LTD), 25. März 1997 (1997-03-25)

## Beschreibung

Die vorliegende Erfindung betrifft eine mikrobiologische Zusammensetzung, ein Verfahren zur Behandlung von Abwässern und/oder Schlämmen und die Verwendung der mikrobiologischen Zusammensetzung.

Man unterscheidet bei Abwässern das sogenannte Schmutzwasser sowie das sogenannte Niederschlagswasser. Schmutzwasser ist das durch häuslichen, gewerblichen, landwirtschaftlichen oder sonstigen Gebrauch in seinen Eigenschaften veränderte und bei Trockenwetter damit abfließende Wasser. Das Niederschlagswasser stammt dagegen von Niederschlägen aus dem Bereich von bebauten oder befestigten Flächen, das abfließt und gesammelt wird. Als Schmutzwasser gelten auch die aus Anlagen zum Behandeln, Lagern und Ablagern von Abfällen austretenden und gesammelten Flüssigkeiten. Häusliche Abwässer fallen aus Spül-, Wasch- und Reinigungsarbeiten sowie aus der Benutzung sanitärer Anlagen an. Die in ihm enthaltenen Stoffe können gelöst, kolloidal oder als Schwimm-, Schweb- und Sinkstoffe vorliegen.

Nach dem Abwasserabgabengesetz (AbwAG) unterscheidet man verschiedene Kategorien von Abwässern. Dabei wird die Schmutzkonzentration im Kommunalabwasser zugrunde gelegt.

Gewerbliches und industrielles Abwasser ist hinsichtlich Art und Konzentration der in ihm enthaltenen Schmutzstoffe stark von der Herkunft abhängig. So enthält Abwasser von Zellstoff- und Zuckerfabriken, Hefefabriken, Brennereien, Gerberein und Seifenfabriken eine hohe Konzentration anorganischen Schmutzstoffen. Im Abwasser der Kalündustrie sowie von Bergbau und von Salinen findet man dagegen großen Mengen an Salzen, Säuren oder Basen.

Das AbwAG regelt die Abgaben für das Einleiten von Abwasser ins Gewässer. Die Genehmigung einer Abwassereinleitung berechtigt nicht ohne weiteres zur kostenlosen Benutzung des Gewässers. Soweit eine Abwassereinleitung über die sogenannte "Vorbelastung des Gewässers" hinausgeht, ist eine Sonderabgabe zu zahlen. Darüber hinaus ist der Verursacher des Abwassers gehalten, für die Reinigung des Abwassers zu sorgen.

Bei der Abwasserbehandlung unterscheidet man zwischen der mechanischen Abwasserbehandlung und der biologischen Abwasserbehandlung. Bei der mechanischen Abwasserbehandlung werden nicht gelöste Stoffe im Abwasser abgetrennt. Grobe Stoffe werden beispielsweise durch Rechen, Sand im Sandfang, aufschwimmende Stoffe, wie Fette und Öle durch Leichtstoffabscheider, sink- oder absetzbare Stoffe (oft nach Neutralisation) im Vorklärbecken zurückgehalten.

Aus der EP 0 372 520 A2 ist beispielsweise ein Verfahren zur Herabsetzung der Schleim- und Belagbildung in Wasserkreisläufen bekannt, bei dem den Kreislaufwässern Mikroorganismen bzw. Mischungen von Mikroorganismen zugegeben werden.

Bei der biologischen Abwasserreinigung werden in der Regel aerobe und anaerobe Bakterien, verwendet. Während die Aerobia mit freiem Sauerstoff leben, benötigen die Anaerobia chemisch gebundenen Sauerstoff. Dabei ist eine Reihe von Bakterien in der Lage, sowohl unter aeroben als auch anaeroben Bedingungen die Abwässer abzubauen. Vorteilhaft bei der aeroben biologischen Abwasserreinigung ist die Verschiedenartigkeit der Bakterienpopulation und die große Breite abbaubarer Substanzen. Nachteile bestehen allerdings darin, dass ein großer Aufwand hinsichtlich der Belüftung sehr groß ist.

während bei der anaeroben biologischen Abwasserreinigung freier Sauerstoff nicht erwünscht ist, muß die Spezifität der Abwasserströme sehr hoch sein. Die ebenfalls hohe Spezifität und Abhängigkeit der Anaerobia untereinander bezüglich ihres Stoffwechsels verlängert die Verweilzeit des Abwassers in einer solchen Anlage.

Die Auflagen zur Abwasserreinigung sind sehr hoch. Es ist auch zu beachten, dass der Verursacher für die Ableitung mit Kosten belastet ist, soweit er nicht, zumindest in einem bestimmten Rahmen, für die Reinigung des Abwassers sorgt.

Mittlerweile gewinnt die biologische Abwasserreinigung neben der mechanischen Abwasserreinigung zunehmend an Bedeutung. Wie bereits eingangs ausgeführt wurde, erfordern die bisher eingesetzten Bakterien, je nach dem, ob sie aerob oder anaerob leben, bestimmte Bedingungen, die in den entsprechenden Abwasserreinigungsanlagen einzuhalten sind. Diese äußerem Umstände sind ohne weiteres von der Großindustrie einzuhalten, indem entsprechend aufwändige Kläranlagen gebaut werden. Es sind auf dem Gebiet der biologischen Abwasserreinigung allerdings noch keine Lösungsmöglichkeiten vorgeschlagen worden, kleineren oder mittleren Betrieben die Möglichkeit zu eröffnen, ihre Abwässer, anorganischer wie organischer Art, zu reinigen, um somit den gesetzlichen Auflagen nachzukommen und damit zugleich die Umwelt zu schützen.

Es ist daher Aufgabe der vorliegenden Erfindung, ein Mittel in der biologischen Abwasserreinigung zur Verfügung zu stellen, das direkt eingesetzt werden kann, ohne dass aufwändige Anlagen zur biologischen Abwasserbehandlung notwendig sind.

Diese Aufgabe wird mit der mikrobiologischen Zusammensetzung gemäß Patentanspruch 1 gelöst.

Die vorliegende Erfindung betrifft eine mikrobiologische Zusammensetzung, die eine Mischung aus photosynthetisch arbeitenden Mikroorganismen und Leuchtbakterien in einer breitbandigen, biologischen Lösung umfaßt.

Die Unteransprüche betreffen bevorzugte Ausführungsformen der erfindungsgemäßen mikrobiologischen Zusammensetzung.

Die vorliegende Erfindung betrifft außerdem ein Verfahren zur Behandlung von Abwässern und/oder Schlämmen, bei dem die Abwässer oder Schlämme vor Eintritt in das kommunale Kanalsystem mit der oben definierten mikrobiologischen Zusammensetzung versetzt werden.

Die Unteransprüche betreffen besondere Ausführungsformen des erfindungsgemäßen Verfahrens.

Des weiteren betrifft die vorliegende Erfindung die Verwendung der oben definierten mikrobiologischen Zusammensetzung zur Reinigung von Abwässern und/oder Schlämmen.

Es hat sich herausgestellt, dass die erfindungsgemäße mikrobiologische Zusammensetzung direkt vor Ort angewendet werden kann. Das bedeutet, dass eine wirksame Menge der mikrobiologischen Zusammensetzung bereits in das Rohrleitungssystem, das in das kommunale Kanalsystem einmündet, eingegeben wird. Somit ist es möglich, ohne apparativen Aufwand bzw. anlagentechnischen Aufwand eine biologische Abwasserreinigung vorzunehmen. Die erfindungsgemäße Zusammensetzung verweilt für eine bestimmte Zeit im Rohrleitungssystem des Abwasserverursachers und reinigt das Abwasser praktisch an dem Ort, an dem es verworfen wird.

Erfindungswesentlich ist, dass die mikrobiologische Mischung zwei Gruppen von Mikroorganismen enthält. Das Wechselspiel zwischen den photosynthetisch arbeitenden Mikroorganismen und den Leuchtbakterien führt dazu, dass die photosynthetisch arbeitenden Mikroorganismen durch die Leuchtbakterien zur Photosynthese angeregt werden. Die Mikroorganismen betreiben die Photosynthese mit Schwefelwasserstoff und Wasser als Edukt und setzen Schwefel bzw. Sauerstoff frei. Ferner können sie Stickstoff sowie Phosphat binden und organische sowie anorganische Materie abbauen.

Bevorzugt werden in der erfindungsgemäßen mikrobiologische Zusammensetzung photosynthetisch arbeitende Mikroorganismen verwendet, die fakultativ phototroph sind. Phototroph fakultativ bedeutet, dass die Mikroorganismen sowohl unter anaeroben Bedingungen im Licht als auch unter aeroben Bedingungen im Dunklen wachsen können.

Zu den Photosynthesebakterien gehören gramnegative aerobe stabförmige und kreisförmige Bakterien sowie grampositive kreisförmige Bakterien. Diese können Endosporen aufweisen oder ohne Sporen vorhanden sein. Dazu zählen beispielsweise auch grampositive Aktinomyceten und verwandte Bakterien.

In diesem Zusammenhang können auch stickstoffbindende Organismen genannt werden. Dazu gehören beispielsweise Algen, wie Anabena Nostoc in Symbiose mit Azola. Des weiteren können Aktinomyceten, z.B. Frankia in Symbiose mit Erlen und Bakterien, wie Rhizobium in Symbiose mit Leguminosen, erwähnt werden.

Außerdem können auch aerobe Algen, Azotobacter, methanoxidierende Bakterien und Schwefelbakterien verwendet werden. Dazu zählen auch grüne Schwefelbakterien und braun-grüne Photosynthesebakterien. Hier können auch nicht violette Schwefelbakterien und violette Schwefelbakterien genannt werden.

Es ist bevorzugt, dass in der erfindungsgemäßen mikrobiologische Zusammensetzung als fakultativ phototrophe Mikroorganismen, Prochlorophyten, Cyanobakterien, grüne Schwefelbakterien, Purpurbakterien, Chloroflexusähnliche Formen und Heliobakterium und Heliobacillus-ähnliche Formen enthalten sind. Die vorgenannten fakultativ phototrophen Mikroorganismen können auch als Mischungen aus zwei oder mehr davon vorliegen. In einer ganz besonderen Ausführungsform liegen alle sechs genannten Mikroorganismen als Mischung vor.

Wie bereits oben ausgeführt wurde, wird die Abwasserreinigung mit der erfindungsgemäßen mikrobiologischen Zusammensetzung im Rohrleitungssystem oder dergleichen des Anwenders durchgeführt. Jedoch kann dort die Photosynthese der photosynthetisch arbeitenden Mikroorganismen nur mit Hilfe einer Lichtquelle funktionieren. Das Licht, das die Photosynthese antreibt, stammt von den Leuchtbakterien, die als zweite essentielle Komponente in der mikrobiologischen Zusammensetzung der vorliegenden Erfindung enthalten sind. Diese Leuchtbakterien besitzen eine Leuchtkraft, d.h. sie sind in der Lage, Lichtquanten auszusenden. Es handelt sich hierbei um ein System, das enzymatisch abläuft. Als Beispiel kann hier das Luciferin-Luciferase-System genannt werden.

In einer bevorzugten Ausführungsform sind in der erfindungsgemäßen Mischung als Leuchtbakterien Photobacterium phosphoreum, Vibrio fischeri, Vibrio harveyi, Pseudomonas lucifera oder Beneckea enthalten. Es ist auch möglich, eine Mischung aus mindestens zwei daraus zu wählen.

Zur Optimierung der erfindungsgemäßen mikrobiologischen Zusammensetzung können weitere Bestandteile darin enthalten sein. Vorzugsweise sind solche Nebenbestandteile Pflanzenextrakte, Enzyme, Spurenelemente, Polysaccharide, Alginderivate, andere Mikroorganismen wie oben. Die Nebenbestandteile können einzeln oder in Kombination in der erfindungsgemäßen mikrobiologischen Zusammensetzung vorliegen. Die Pflanzenextrakte können beispielsweise Spitzwegerich enthalten.

Es hat sich herausgestellt, dass es günstig ist, Enzyme hinzuzufügen, wie beispielsweise Lipasen.

Als Spurenelemente kann jedes Spurenelement enthalten sein, das die Abwasserreinigung günstig beeinflußt. Hier können beispielsweise Natrium, Mangan, Kupfer, Bor, Zink, Eisen, Schwefel, Magnesium, Kalium, Calcium, Phosphat oder Mischungen daraus genannt werden.

Es kann auch jedes Polysaccharid zugesetzt werden, das für die Wirksamkeit der vorliegenden mikrobiologischen Zusammensetzung günstig ist. Hierzu zählen beispielsweise Roh- oder Zuckerrübenmelasse.

Als Alginderivate werden bevorzugt Alginate eingesetzt. Die Alginate dienen zur O₂-Produktion.

Als weitere Mikroorganismen kann jeder Mikroorganismus in Frage kommen, der eine positive Wirkung auf die erfindungsgemäße mikrobiologische Zusammensetzung ausübt. Bevorzugt werden Milchsäurebakterien eingesetzt. Es ist allerdings auch möglich, sogenannte Eukomycota zuzusetzen, wie beispielsweise Mastigomycotina, Zygomycotina, Basidiomycotina und/oder Deuteromycotina.

In der Regel weist die mikrobiologische Zusammensetzung einen pH-Wert im Bereich von 3,4 bis 4,2 auf. Ein beverzugter pH Wertbereich liegt zwischen 3,4 bis 3,8.

Als Nährlösung für die erfindungsgemäße mikrobiologische Zusammensetzung wird im allgemeinen eine Lösung verwendet, die dazu beiträgt, dass die darin enthaltenen Bestandteile, insbesondere die Mikroorganismen, ohne weiteres darin leben können. Dabei kommt es insbesondere darauf an, dass die Wechselwirkung der Photosynthesebakterien und der Leuchtbakterien vollständig zum Tragen kommt. Es hat sich erwiesen, dass eine biologische Nährlösung mit Melasse, insbesondere Rohzuckermelasse oder Zuckerrübenmelasse als Hauptbestandteil geeignet ist.

Die photosynthetisch arbeitenden Mikroorganismen und die. Leuchtebakterien liegen in der erfindungsgemäßen mikrobiologischen Zusammensetzung normalerweise in einem Verhältnis von 1 : 10 bis 1 : 500 vor. Ein bevorzugtes Verhältnis ist 1 : 100.

Das erfindungsgemäße Verfahren zur Behandlung von Abwässern und/oder Schlämmen zeichnet sich dadurch aus, dass es vom Verursacher selbst durchgeführt werden kann, ohne dass aufwendige Anlagen vorhanden sein müssen, die normalerweise bei der biologischen Abwasseraufbereitung notwendig sind.

Das Verfahren wird durchgeführt, indem die Abwässer und/oder Schlämme vor Eintritt in das kommunale Kanalsystem mit der oben definierten mikrobiologischen Zusammensetzung versetzt werden.

Die einzusetzende Menge hängt davon ab, wie stark das Abwasser verschmutzt ist. So kann es ausreichen, ein Liter pro Woche in den Ausguß zu geben, während bei extremer Verschmutzung bis zu 15 Liter in der Woche notwendig sind.

Das erfindungsgemäße Verfahren läßt sich hervorragend zur Reinigung von Abwässern und/oder Schlämmen im Bereich der chemischen und pharmazeutischen Industrie, der lebensmittel- und fleischverarbeitenden Industrie, der Gastronomie und des Hotelgewerbes anwenden. Die gereinigten Abwässer und/oder Schlämme können erfindungsgemäß geruchsfrei und umweltunbedenklich in das kommunale Abwassersystem abfließen.

Mit der erfindungsgemäßen mikrobiologischen Zusammensetzung sowie mit dem erfindungsgemäßen Verfahren kann effektiv der Geruch der Abwässer reduziert, wenn nicht sogar eliminiert werden. Das geschieht durch Adsorption und Neutralisation. Des weiteren hat sich herausgestellt, dass insbesondere fette und fettähnliche Substanzen gespalten, gelöst und abgebaut werden können.

Wie bereits oben ausgeführt wurde, kann die erfindungsgemäße mikrobiologische Zusammensetzung auf vielen Gebieten eingesetzt werden. Zu der chemischen und pharmazeutischen Industrie zählen beispielsweise die Entsorgungsindustrie (Entsorgung jeglicher Abfälle) und solche Betriebe, in denen Chemikalien toxischer Art anfallen. Hier können beispielsweise solche Betriebe, wie Lackierereien genannt werden. Im Hüttenwesen fallen oftmals insbesondere dicke anorganische Schlämme an, die ebenfalls erfolgreich mit der erfindungsgemäßen Zusammensetzung behandelt werden können.

In der lebensmittel- und fleischverarbeitenden Industrie eignet sich das erfindungsgemäße Produkt insbesondere für Metzgereien und Molkereien.

In der Gastronomie sind hier insbesondere Restaurants, Gaststätten und Großküchen zu nennen.

Mit der erfindungsgemäßen mikrobiologischen Zusammensetzung und dem erfindungsgemäßen Verfahren zur Reinigung von Abwässern und/oder Schlämmen wird eine schonende Abwasserbehandlung durchgeführt. Es wurde keine Bildung toxischer Nebenprodukte beobachtet. Das mikrobiologisch gereinigte Abwasser kann ohne weiteres in das kommunale Abwassersystem eingeleitet werden.

Die vorliegende Erfindung ist eine alternative Lösung zur beispielsweise Deponierung, Verbrennung und/oder Verwertung von Abfällen und toxischen Substanzen. Gleichzeitig werden dabei organische Reststoffe in Ausgüssen und Rohrleitungen abgebaut. Ein weiterer Vorteil liegt darin, dass die Abwässer erfolgreich hinsichtlich ihrer störenden Gerüche behandelt werden, so dass die Geruchsemission wesentlich herabgesetzt ist, wenn nicht sogar völlig eliminiert ist.

## Patentansprüche

1. Mikrobiologische Zusammensetzung, die eine Mischung aus photosynthetisch arbeitenden, fakultativ phototrophen Mikroorganismen und Leuchtbakterien in einer breitbandigen, biologischen Lösung umfasst, wobei in der Mischung als fakultativ phototrophe Mikroorganismen Prochlorophyten, Cyanobakterien, grüne Schwefelbakterien, Purpurbakterien, Chloroflexusähnliche Formen und Heliobakterium und Heliobacillusähnliche Formen sowie Mischungen aus zwei oder mehr daraus und als Leuchtbakterien Photobacterium phosphoreum, vibrio fischeri, vibrio harveyi, Pseudomonas lucifera oder Beneckea oder Mischungen aus mindestens zwei daraus enthalten sind.

2. Mikrobiologische Zusammensetzung nach Anspruch 1, **dadurch gekennzeichnet, dass** sie weiterhin als Nebenbestandteile Pflanzextrakte, Enzyme, Spurenelemente, Polysaccharide, Alginderivate, andere Mikroorganismen, entweder einzeln oder in Kombination, enthält.

3. Mikrobiologische Zusammensetzung nach Anspruch 2, **dadurch gekennzeichnet, dass** als Enzyme Lipasen enthalten sind.

4. Mikrobiologische Zusammensetzung nach Anspruch 2, **dadurch gekennzeichnet, dass** die Spurenelemente Natrium, Mangan, Kupfer, Bor, Zink, Eisen, Schwefel, Magnesium, Kalium, Calcium, Phosphat oder Mischungen daraus sind.

5. Mikrobiologische Zusammensetzung nach Anspruch 2, **dadurch gekennzeichnet, dass** als Polysaccharide insbesondere Roh- oder Zuckerrübenmelässe enthalten sind.

6. Mikrobiologische Zusammensetzung nach Anspruch 2, **dadurch gekennzeichnet, dass** als Alginderivate Alginate enthalten sind.

7. Mikrobiologische zusammensetzung nach Anspruch 2, **dadurch gekennzeichnet, dass** als ein weiterer Mikroorganismus Milchsäurebakterien enthalten sind.

8. Mikrobiologische Zusammensetzung nach mindestens einem der vorangegangenen Ansprüche, **dadurch gekennzeichnet, dass** sie einen pH-Wert im Bereich von 3,4 bis 4,2 aufweist.

9. Mikrobiologische Zusammensetzung nach Anspruch 8, **dadurch gekennzeichnet, dass** der pH-Wert im Bereich von 3,4 bis 3,8 liegt.

10. Mikrobiologische Zusammensetzung nach mindestens einem der vorangegangenen Ansprüche, **dadurch gekennzeichnet, dass** die photosynthetisch arbeitenden Mikroorganismen und die Leuchtbakterien in einem Verhältnis von 1 : 10 bis 1 : 500, vorzugsweise 1 : 100 enthalten sind.

11. Mikrobiologische Zusammensetzung nach mindestens einem der vorangegangenen Ansprüche, **dadurch gekennzeichnet, dass** die Konzentration der Bakterien im Bereich von 2 - 3 % liegt.

12. Verfahren zur Behandlung von Abwässern und/oder Schlämmen mit den Schritten:
a) Versetzen der Abwässer oder Schlämme mit einer mikrobiologischen Zusammensetzung nach mindestens einem der Ansprüche 1 bis 11;
b) Einleiten der versetzten Abwässer in das kommunale Kanalsystem.

13. Verfahren nach Anspruch 12, **dadurch gekennzeichnet, dass** die Zusammensetzung in einer Menge von 1 1/Woche bis 15 1/Woche hinzugegeben wird.

14. Verfahren nach Anspruch 12 oder 13 zur Reinigung von Abwässern und/oder Schlämmen im Bereich der chemischen und pharmazeutischen Industrie, des Hüttenwesens, der Landwirtschaft, der lebensmittel- und fleischverarbeitenden Industrie, der Gastronomie und des Hotelgewerbes.

15. Verfahren nach Anspruch 14, **dadurch gekennzeichnet, dass** die gereinigten Abwässer und/oder Schlämme geruchsfrei und umweltunbedenklich in das kommunale Abwassersystem abfließen.

16. Verwendung einer mikrobiologischen Zusammensetzung nach mindestens einem der Ansprüche 1 bis 11 zur Reinigung von Abwässern und/oder Schlämmen.

17. Verwendung nach Anspruch 16 für die chemische und pharmazeutische Industrie, das Hüttenwesen, die Landwirtschaft, die lebensmittel- und fleischverarbeitende Industrie, die Gastronomie und das Hotelgewerbe.

## Claims

1. A microbiological composition, which comprises a mixture of photosynthetically active, facultatively phototropic microorganisms and luminescent bacteria in a broad-spectrum biological solution, wherein as facultatively phototropic microorganisms the mixture contains prochlorophytes, cyano bacteria, green sulphur bacteria, purple bacteria, Chloroflexus-like forms and Heliobacterium and Heliobacillus-like forms and also mixtures of two or more thereof, and as luminescent bacteria Photobacterium phosphoreum, Vibrio fischeri, Vibrio harveyi, Pseudomonas lucifera or Beneckea or mixtures of at least two thereof.

2. A microbiological composition according to claim 1, **characterised in that** as minor constituents it furthermore contains plant extracts, enzymes, trace elements, polysaccharides, alginic derivates, other microorganisms, either alone or in combination.

3. A microbiological composition according to claim 2, **characterised in that** lipases are contained as enzymes.

4. A microbiological composition according to claim 2, **characterised in that** the trace elements are sodium, manganese, copper, boron, zinc, iron, sulphur, magnesium, potassium, calcium, phosphate or mixtures thereof.

5. A microbiological composition according to claim 2, **characterised in that** raw molasses or sugar beet molasses are contained as polysaccharides.

6. A microbiological composition according to claim 2, **characterised in that** alginates are contained as alginic derivatives.

7. A microbiological composition according to claim 2, **characterised in that** lactic acid bacteria are contained as a further microorganism.

8. A microbiological composition according to at least one of the preceding claims, **characterised in that** it has a pH value in the range from 3.4 to 4.2.

9. A microbiological composition according to claim 8, **characterised in that** the pH value lies in the range from 3.4 to 3.8.

10. A microbiological composition according to at least one of the preceding claims, **characterised in that** the photosynthetically active microorganisms and the luminescent bacteria are contained in a ratio of 1 : 10 to 1 : 500, preferably 1 : 100.

11. A microbiological composition according to at least one of the preceding claims, **characterised in that** the concentration of the bacteria is in the range from 2 -3%.

12. A method for treating effluents and/or sludges comprising the steps:
a) adding a microbiological composition according to at least one of claims 1 to 11 to the effluents or sludges;
b) introducing the charged effluents into the municipal sewage system.

13. A method to claim 12, **characterised in that** the composition is added in an amount of 1 I/week to 15 1/week.

14. A method to claim 12 or 13 for cleaning effluents and/or sludges in the chemical and pharmaceutical industry, in metallurgy, agriculture, the food and meat processing industry, the catering and hotel industry.

15. A method to claim 14, **characterised in that** the cleaned effluents and/or sludges flow into the municipal sewage system without odour and without harm to the environment.

16. A use of a microbiological composition according to at least one of claims 1 to 11 for cleaning effluents and/or sludges.

17. A use according to claim 16 for the chemical and pharmaceutical industry, metallurgy, agriculture, the food and meat processing industry, the catering and hotel industry.

## Revendications

1. Composition microbiologique comprenant un mélange de microorganismes agissant de manière photosynthétique, éventuellement phototrophes et de bactéries luminescentes dans une solution biologique à large spectre, dans laquelle sont contenus comme microorganismes éventuellement phototrophes, des prochlorophytes, des cyanobactéries, des bactéries du soufre vertes, des bactéries pourpres, des formes de type *Chloroflexus* et des héliobactéries et des formes de type héliobacilles ainsi que des mélanges de deux ou de plusieurs d'entre eux, et comme bactéries luminescentes, *Phosphobacterium phosphoreum, Vibrio fischeri, Vibrio harveyi, Pseudomonas lucifera* ou *Beneckea* ou des mélanges de deux d'entre eux.

2. Composition microbiologique selon la revendication 1, **caractérisée en ce qu'**elle contient en outre comme composants minoritaires, des extraits végétaux, des enzymes, des oligo-éléments, des polysaccharides, des dérivés d'algine, d'autres microorganismes, seuls ou en combinaison.

3. Composition microbiologique selon la revendication 3, **caractérisée en ce qu'**elle contient, comme enzyme, des lipases.

4. Composition microbiologique selon la revendication 2, **caractérisée en ce que** les oligo-éléments sont le sodium, le manganèse, le cuivre, le bore, le zinc, le fer, le soufre, le magnésium, le potassium, le calcium, le phosphate ou leurs mélanges.

5. Composition microbiologique selon la revendication 3, **caractérisée en ce qu'**elle contient, comme polysaccharides, en particulier de la mélasse brute ou de la mélasse de betterave à sucre.

6. Composition microbiologique selon la revendication 2, **caractérisée en ce qu'**elle contient comme dérivés d'algine, des alginates.

7. Composition microbiologique selon la revendication 3, **caractérisée en ce qu'**elle contient comme autre microorganisme, des bactéries lactiques.

8. Composition microbiologique selon au moins l'une quelconque des revendications précédentes, **caractérisée en ce qu'**elle présente un pH dans la plage de 3,4 à 4,2.

9. Composition microbiologique selon la revendication 8, **caractérisée en ce qu'**elle présente un pH dans la plage de 3,4 à 3,8.

10. Composition microbiologique selon au moins l'une quelconque des revendications précédentes, **caractérisée en ce que** les microorganismes agissant de manière photosynthétique et les bactéries luminescentes sont comprises en un rapport de 1:10 à 1:500, de préférence 1:100.

11. Composition microbiologique selon au moins l'une quelconque des revendications précédentes, **caractérisée en ce que** la concentration en bactéries se situe dans une plage de 2 à 3 %.

12. Procédé de traitement des eaux usées et/ou des boues, comprenant les étapes consistant à :
a) modifier les eaux usées ou les boues avec une composition microbiologique selon au moins l'une quelconque des revendications 1 à 11 ;
b) introduction des eaux usées modifiées dans le réseau communal.

13. Procédé selon la revendication 12, **caractérisé en ce que** la composition est ajoutée en une quantité de 1 l/semaine jusqu'à 15 l/semaine.

14. Procédé selon la revendication 12 ou 13 pour la purification des eaux usées et/ou des boues dans le domaine de l'industrie chimique et pharmaceutique, de la métallurgie, de l'agriculture, de l'industrie agroalimentaire et de transformation de la viande, de la gastronomie et de l'hôtellerie.

15. Procédé selon la revendication 14, **caractérisé en ce que** les eaux usées et/ou boues purifiées sont dépourvues d'odeurs et peuvent s'écouler sans danger pour l'environnement, dans le système d'évacuation communal .

16. Utilisation d'une composition microbiologique selon au moins l'une quelconque des revendications 1 à 11 pour la purification des eaux usées et/ou des boues.

17. Utilisation selon la revendication 16, pour l'industrie chimique et pharmaceutique, la métallurgie, l'agriculture, l'industrie agroalimentaire et de transformation de la viande, la gastronomie et l'hôtellerie.
